# EUROPÄISCHE PATENTSCHRIFT

(11) **EP 1 071 484 B1**
(45) Veröffentlichungstag und Bekanntmachung des Hinweises auf die Patenterteilung: **04.10.2001**
(21) Anmeldenummer: 99927712.2
(22) Anmeldetag: 21.04.1999
(51) Int. Cl.: A61L 27/00

(54) **VERWENDUNG EINER AUTOPOLYMERISIERENDEN ZUSAMMENSETZUNG AUF ORGANOSILOXANBASIS**
UTILIZATION OF AN AUTOPOLYMERIZING ORGANOSILOXANE-BASED COMPOUND
UTILISATION D'UNE COMPOSITION AUTOPOLYMERISABLE A BASE D'ORGANOSILOXANE

(30) Priorität: 22.04.1998 DE 19817698
(43) Veröffentlichungstag der Anmeldung: 31.01.2001
(73) Patentinhaber: Zöllner, Jan, 65307 Bad Schwalbach (DE); Eysel, Peer, 63505 Langenselbold (DE)
(72) Erfinder: Zöllner, Jan, 65307 Bad Schwalbach (DE); Eysel, Peer, 63505 Langenselbold (DE)
(74) Vertreter: Weber, Dieter, Dr.
(86) Internationale Anmeldenummer: DE9901215
(87) Internationale Veröffentlichungsnummer: WO9953970

(56) Entgegenhaltungen:
- DE-A- 3 911 610
- FR-A- 2 694 882
- US-A- 3 867 728
- US-A- 5 248 706

## Beschreibung

Bereits ab dem dreißigsten Lebensjahr sind bei jedem Menschen degenerative Veränderungen der Bandscheibe nachzuweisen. Bei Bandscheibenvorfällen werden operative Eingriffe vorgenommen, die aber im Bereich der Lendenwirbelsäule von einer zum Teil hohen Mißerfolgsrate geprägt sind. Die Rate der Rezidivbandscheibenvorfälle und des sogenannten Failed Back Surgery Syndrom oder Postnucleotomiesyndroms ist relativ hoch. Dabei treten nach Nukleotomie Schmerzen auf, die häufig nach beschwerdefreiem Intervall in Erscheinung treten. Die zunehmende Degeneration des operierten Segments und die Degeneration der Bogengelenke werden als wesentliche Ursachen für ein sich verschlechterndes Langzeitergebnis angesehen. Dies bedeutet, daß eine der wichtigsten Ursachen der Beschwerden nach Bandscheibenoperationen die vermehrte Mobilität des Bewegungssegmentes darstellt. Eine mögliche Lösung ist die Versteifung des Bewegungssegmentes mit dem Risiko der vermehrten Belastung der angrenzenden Segmente und der Gefahr der Anschlußinstabilität. Eine andere Möglichkeit besteht in dem künstlichen Bandscheibenersatz beispielsweise mit Hilfe eines hydrophilen Materials in einem Polyethylennetz eingebunden, das im trockenen Zustand implantiert wird.

Versuche mit einem Wirbelsäulenmodell ergaben, daß eine wesentliche Ursache für ein sich verschlechterndes Langzeitergebnis nach Bandscheibenoperationen die vermehrte Mobilität des Bewegungssegmentes ist. Die der Erfindung zugrundeliegende Aufgabe bestand somit darin, Bandscheibenimplantate zu bekommen, die das physiologische Bewegungsausmaß der Bandscheibe so gut wie möglich wiederherstellen. Diese Aufgabe wird erfindungsgemäß gelöst, indem man eine flüssige oder pastenartige, bei Umgebungstemperatur zu dauerelastischem Silicon autopolymerisierende Zusammensetzung auf Organosiloxanbasis zur Herstellung von Bandscheibenimplantaten verwendet.

Die Bandscheibe besteht aus einer Außenzone, dem Anulus fibrosus, mit den sogenannten Sharpeyschen Fasern und einer gallertartigen parachondralen Innenzone, dem Nucleus pulposus. Erfindungsgemäß wird die autopolymerisierende Zusammenetzung auf Organosiloxanbasis vorzugsweise zur Herstellung von Implantaten lediglich des Nucleus pulposus verwendet, während der Anulos fibrosus unverändert erhalten bleibt. Da die erfindungsgemäß verwendete Zusammensetzung flüssig oder pastenartig ist, kann diese Zusammensetzung nach Entfernung der gallertartigen Innenzone aus dem Anulus fibrosus in den entstandenen Hohlraum eingeführt werden und unterliegt dort der Autopolymerisation. Der Anulus fibrosus dient also dabei einerseits dazu, den Abstand der Wirbelsegmente zu halten, andererseits aber gleichzeitig als Form für die flüssige oder pastenförmige Zusammensetzung nach der Erfindung, die in den Innenraum dieser Form eingespritzt wird. Hierzu genügt ein endoskopisch minimal invasiver Zugang zur Bandscheibe über den retroperitonealen Raum von ventral oder über den standardisierten Zugang von dorsal. Nach dem Ausräumen des Nucleus pulposus und Einbringen der hydrophoben Zusammensetzung auf Organosiloxanbasis nach der Erfindung kann der Anulus fibrosus anschließend verschlossen werden. Die Zusammensetzung polymerisiert ohne zusätzliche Erwärmung zu einem dauerelastischen Silicon unter kompletter Ausfüllung des Hohlraumes innerhalb des Anulus fibrosus, wobei eine maximale Fläche für die Lastübertragung genutzt werden kann, so daß ein gleichmäßiges Bewegungsbild entsteht. Mit der Erfindung kann daher die segmentale Stabilität des nukleotomierten Bandscheibensegmentes wiederhergestellt werden.

Die erfindungsgemäß verwendete Zusammensetzung auf Organosiloxanbasis ist in sterilisiertem Zustand zu verwenden. Da eine solche Sterilisierung im Regelfall nicht vom Verwender der flüssigen oder pastenförmigen Zusammensetzung durchgeführt werden kann, ist es zweckmäßig, die Zusammensetzung werkseitig zu sterilisieren und in einer innensterilen Verpackung in den Handel zu bringen.

Die erfindungsgemäß verwendeten Zusammensetzungen müssen neben möglichst hoher Dauerbelastungsfähigkeit eine möglichst gute Biokompatibilität haben. Es zeigte sich, daß Zusammensetzungen auf Polydimethylsiloxanbasis bei der Autopolymerisation Silicone ergeben, die beide Eigenschaften besitzen. Da die Bandscheibe erheblichen Druckbelastungen ausgesetzt ist, war dieses Ergebnis überraschend und nicht vorhersehbar. Die aus Polydimethylsiloxanen erhaltenen dauerelastischen Silicone haben keinen nachteiligen Einfluß auf das sie umgebende Gewebe, auf die Zellausbreitung und normale Zellteilungsaktivität. Degenerative Veränderungen wurden nicht festgestellt. Insbesondere sind Zusammensetzungen auf der Basis linearer Polydimethylsiloxane von Vorteil.

Durch Copolymerisation der Methylsiloxane mit anders substituierten Siloxanen können die Reaktionsdauer bei der Autopolymerisation und die Eigenschaften der erhaltenen Silicone variiert werden. Beispielsweise können statt Methylgruppen Phenylreste, Ethylgruppen oder Vinylreste eingebaut werden, so daß beispielsweise gemischte Methylphenyl-, Ethylmethyl- oder Methylvinylsiloxane entstehen. Bei solchen Copolymerisaten können die Substituenten willkürlich oder als Blockcopolymere angeordnet werden. Die Endgruppen der Polydimethylsiloxane sind vorzugsweise Trimethylsiloxygruppen, können aber wenigstens teilweise auch Silanolgruppen, Vinylgruppen oder Hydridendgruppen sein. Die Viskosität der linearen Polydimethylsiloxane kann durch Kettenabbruchgruppen gesteuert werden, was für den Erfindungsgegenstand wesentlich ist, da die Zusammensetzungen bei der Verwendung flüssig bis pastenartig sein sollen.

Um bei Umgebungstemperatur autopolymerisierend zu sein, enthalten die erfindungsgemäß verwendeten Zusammensetzungen einen Katalysator. Bekannte Siloxankatalysatoren sind Amine, wie Aminopropylsilanderivate, und Carbonsäuresalze von Blei, Zinn und Zink sowie organische Salze von Eisen, Cadmium, Barium, Antimon und Zirkonium. Zinnoctoate, -laurate und -oleate sowie Salze von Dibutylzinn sind besonders brauchbar. Unter den Katalysatoren müssen allerdings solche ausgewählt werden, die biologisch verträglich sind. Hierzu gehören insbesondere additionskatalysierende Edelmetallkomplexe der Gruppe VIII, wie von Platin, Rhodium und Ruthenium, die in sehr kleinen Mengen, wie in Konzentrationen so niedrig wie 1 bis 2 ppm, die Autopolymerisation in annehmbaren Polymerisationszeiten katalysieren. Insbesondere sind Platinkatalysatoren, wie Platin-Olefinkomplexe, brauchbar, eine Addition von Si-H-Endgruppen mit Olefinen, wie vinylfunktionellen Siloxanen zu katalysieren. Bevorzugte Zusammensetzungen, die zu dauerelastischen Siliconen polymerisieren, werden daher von Gemischen linearer hydridfunktioneller Polydimethylsiloxane und linearer vinylfunktioneller Polydimethylsiloxane im Gemisch mit einem geeigneten Katalysator, insbesondere einem Platinkatalysator, wie Chlorplatinsäure oder einer anderen Platinverbindung, gebildet. Um eine vorzeitige Polyaddition zu verhindern, werden solche Zusammensetzungen in zwei getrennten Packungen gelagert und erst unmittelbar vor der Verwendung miteinander vereinigt. Gewöhnlich wird der Katalysator zusammen mit den vinylfunktionellen Polydimethylsiloxanen in einer Verpackung und das hydridfunktionelle Polydimethylsiloxan, gegebenenfalls mit einem üblichen Härtungsmittel, gegebenenfalls unter Zugabe von vinylfunktionellem Polydimethylsiloxan, aber ohne Katalysator, in der zweiten Verpackung aufbewahrt. Die verwendeten Mengenverhältnisse der beiden Polydimethylsiloxane hängen von den erwünschten Eigenschaften des dauerelastischen Silicons ab.

Gegebenenfalls kommt als Katalysator auch Luftfeuchtigkeit in Betracht, doch ist dies wegen der erforderlichen Sterilität der Zusammensetzung bei der erfindungsgemäßen Anwendung weniger bedeutsam.

Das mittlere Molekulargewicht (Zahlenmittel) der erfindungsgemäß verwendeten Zusammensetzungen liegt im Bereich von 1000 bis 150 000, zweckmäßig im Bereich von 50 000 bis 100 000.

Die erfindungsgemäß verwendeten Zusammensetzungen können zusätzlich übliche Füllstoffe, wie feinteilige Kieselsäuren, wie Rauchkieselsäure, enthalten, wobei solche Füllstoffe Teilchengrößen von maxial 1 *µ*m, wie beispielsweise im Bereich von 0,01 bis 0,5 *µ*m, haben sollten. Andere in Betracht kommende Füllstoffe sind dehydrierte Kieselgele, Diatomeenerde, feinteiliges Titandioxid, Aluminiumoxid oder Zirkonoxid. Im übrigen können in die erfindungsgemäß verwendeten Zusammensetzungen bei Organosiloxanmassen übliche Hilfsstoffe eingearbeitet werden, sofern diese biokompatibel sind und die erwünschten Eigenschaften des auspolymerisiserten Silicons, insbesondere die Dauerelastizität und Druckbeständigkeit, nicht beeinträchtigen.

## Patentansprüche

1. Verwendung einer flüssigen oder pastenartigen, bei Umgebungstemperatur zu dauerelastischem Silicon autopolymerisierenden Zusammensetzung auf Organosiloxanbasis zur Herstellung von Bandscheibenimplantaten.

2. Verwendung nach Anspruch 1 zur Herstellung von Nucleus pulposus-Implantaten ist.

3. Verwendung nach Anspruch 1 oder 2, worin die Zusammensetzung eine Zusammensetzung auf Polydimethylsiloxanbasis ist.

4. Verwendung nach einem der Ansprüche 1 bis 3, worin die Zusammensetzung eine Zusammensetzung mit einem Gehalt eines Platinkatalysators ist.

5. Verwendung nach einem der Ansprüche 1 bis 4, worin die Zusammensetzung eine katalysatorhaltige Zusammensetzung auf der Basis hydridfunktioneller Siloxane und vinylfunktioneller Siloxane ist.

6. Verwendung nach einem der Ansprüche 1 bis 5, worin die Zusammensetzung in sterilisierter Form vorliegt.

## Claims

1. Use of a liquid or pasty organo siloxane-based composition autopolymerizing to a permanently elastic silicone at ambient temperature, for the production of intervertebral disk implants.

2. Use according to claim 1 for the production of nucleus pulposus implants.

3. Use according to claim 1 or 2, wherein the composition is a polydimethylsiloxane based composition.

4. Use according to one of the claims 1 to 3, wherein the composition is a composition containing a platinum catalyst.

5. Use according to one of the claims 1 to 4, wherein a catalyst containing composition based on hydride functional siloxanes and vinyl functional siloxanes.

6. Use according to one of the claims 1 to 5, wherein the composition exists in sterilized form.

## Revendications

1. Utilisation d'une composition fluide ou de type pâte à base d'organosiloxane, autopolymérisant à température ambiante en silicone à élasticité permanente, pour fabriquer des implants pour disque intervertébral.

2. Utilisation selon la revendication 1 pour fabriquer des implants pour noyau gélatineux (nucleus pulposus).

3. Utilisation selon la revendication 1 ou 2, la composition étant une composition à base de polydiméthylsiloxane.

4. Utilisation selon l'une des revendications 1 à 3, la composition étant une composition présentant une teneur en catalyseur au platine.

5. Utilisation selon l'une des revendications 1 à 4, la composition étant une composition contenant un catalyseur à base de siloxane à fonctionnalité hydrure et de siloxane à fonctionnalité vinyle.

6. Utilisation selon l'une des revendications 1 à 5, la composition se présentant sous une forme stérilisée.
